# EUROPEAN PATENT APPLICATION

(11) **EP 3 417 909 A1**
(43) Date of publication of application: **26.12.2018**
(21) Application number: 16901081.6
(22) Date of filing: 03.05.2016
(51) Int. Cl.: A61N 5/06, A61B 6/04, A61N 5/00

(54) **CAPSULE-TYPE PHOTOTHERAPY APPARATUS**

(71) Applicant: Kumgang Advance Co., Ltd., Wonju-si, Gangwon-do 26354 (KR)
(72) Inventor: JUNG, Sang Sik, Wonju-si Gangwon-do 26367 (KR)
(74) Representative: MERH-IP Matias Erny Reichl Hoffmann Patentanwälte PartG mbB
(86) International application number: PCT/KR2016/004677
(87) International publication number: WO 2017/191854

(57) **Abstract**

The present invention relates to a capsule-type phototherapy apparatus and, more particularly, to a capsule-type phototherapy apparatus which can accommodate a user therein and perform a far-infrared and a visible light procedure on the user's body. The present invention comprises: a bed in which a heat source is installed and on which a user lies; and a cover which has a visible light irradiation unit installed therein and is rotatably installed to one side of the bed so as to open and close the bed.

## Description

### [Technical Field]

The present invention relates to a capsule-type phototherapy apparatus and, more particularly, to a capsule-type phototherapy apparatus which can accommodate a user therein and perform far-infrared and visible light treatment procedure on the user's body.

### [Background Art]

Light treatment, i.e. phototherapy, refers to a treatment procedure entailing irradiating a part of the human body with natural or artificial light to treat disease, and is known to be effective for treatment of various stress symptoms, depression, insomnia, emotional disorders, diseases such as skin disease, autonomic regulation, immunity improvement, or the like, and further has a wide range of therapeutic effects such as treatment of wounds, skin cancer treatment, etc.

If selecting appropriate wavelength and intensity of light in consideration of the nature of disease and emitting the light, photons are transferred or absorbed through skin and activate a mechanism of the body to thus treat the disease.

However, a conventional phototherapy apparatus has a relatively small size and emits light in only a single direction. For this reason, upon conducting a treatment procedure, several phototherapy devices are required to emit light to different portions of the body of a patient, hence causing discomfort and high electricity consumption.

### [Disclosure]

### [Technical Problem]

In order to solve the above problems, an object of the present invention is to provide a capsule-type phototherapy apparatus which can accommodate a user inside the apparatus and execute a far-infrared and visible light treatment procedure on the body of the user.

### [Technical Solution]

In order to accomplish the above object, the present invention provides a capsule-type phototherapy apparatus, including: a bed in which a heat source is installed, wherein a user can lie on the top of the bed; and a bed cover in which a visible light emission device is installed and which is rotatably installed at one side of the bed so as to open and close the bed.

### [Advantageous effects]

The capsule-type phototherapy apparatus and the visible light emission device provided therein according to the present invention may attain effects of performing a treatment procedure ('procedure') based on far-infrared and visible light on the body of a user through a heat source as well as the visible light emission device.

Further, it is possible to prevent bacteria, mold and dust from propagating over air to the outside through the bed and cover, and to inject fresh oxygen into the apparatus, thereby implementing a clean room.

In addition, constant temperature and humidity, shower, dry-wet type sauna, music therapy and aromatherapy functions are accomplished so that the user can receive the procedure in a pleasant environment.

### [Description of Drawings]

FIG. 1 is a perspective view of a capsule-type phototherapy apparatus according to an embodiment of the present invention, wherein an upper cover is detached from the bed while a lower cover is open.
FIG. 2 is a front view of the capsule-type phototherapy apparatus shown in FIG. 1.
FIG. 3 is a plan view of the bed shown in FIG. 1.
FIG. 4 is a side view of the capsule-type phototherapy apparatus shown in FIG. 1.
FIG. 5 is a conceptual diagram illustrating a visible light emission device in the capsule-type phototherapy apparatus shown in FIG. 1.
FIG. 6 is a block diagram illustrating the visible light emission device shown in FIG. 5.
FIG. 7 illustrates the visible light emission device shown in FIG. 5.
FIGS. 8 and 9 are schematic views of a cover part shown in FIG. 5.
FIG. 10 is a block diagram illustrating MODE in a display unit shown in FIG. 5.
FIG. 11 illustrates a time setting screen of the display unit shown in FIG. 5.
FIG. 12 is a flowchart illustrating a visible light emission process by the visible light emission device shown in FIG. 5.

### [Best mode]

Hereinafter, in order to concretely describe the present invention sufficiently to easily execute technical spirits of the present invention by persons having common knowledge in the technical field to which the present invention pertains (referred to as 'those skilled in the art'), most preferred embodiments of the present invention will be described in detail with reference to the accompanying drawings.

First, with regard to reference numerals denoting components of each drawing, it should be noted that the same components will have the same reference numerals as much as possible even though shown in different drawings.

Further, in the following description of the present invention, when a detailed description of known functions and configurations relevant to the present invention are determined to obscure the gist of the present invention, the above description will be omitted.

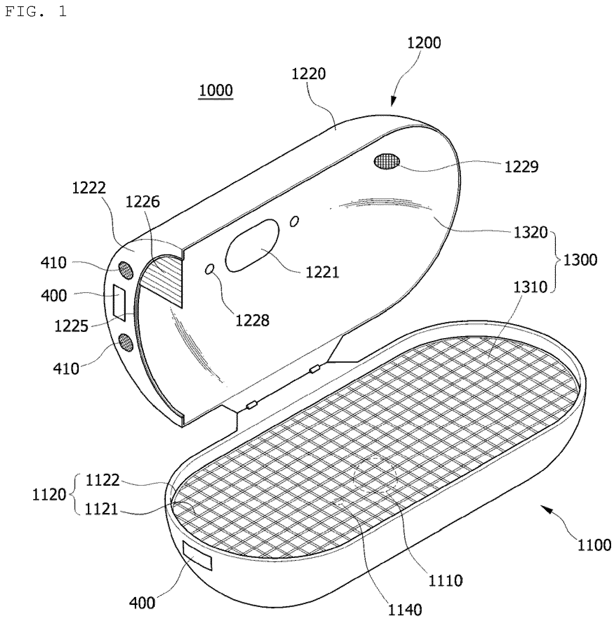

Before concretely describing the present invention, with regard to directions represented in the following description, the term 'upper' in the upper portion is defined as a direction toward the cover 200 relative to the bed 100 shown in FIG. 1, while the term 'lower' in the lower portion is defined as a direction toward the bed 100 relative to the cover 200.

Hereinafter, referring to FIGS. 1 to 3, the capsule-type phototherapy apparatus according to one embodiment of the present invention will be described in detail.

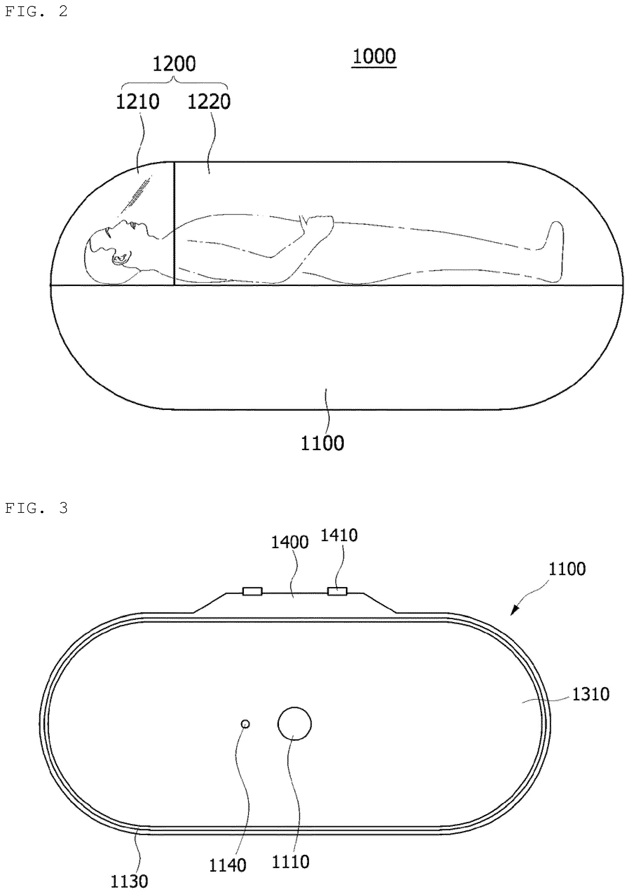

FIG. 1 is a perspective view of a capsule-type phototherapy apparatus according to one embodiment of the present invention, wherein an upper cover 1210 is detachable from a bed 1100 while a lower cover 1220 is open. FIG. 2 is a front view of the capsule-type phototherapy apparatus 1000 shown in FIG. 1.

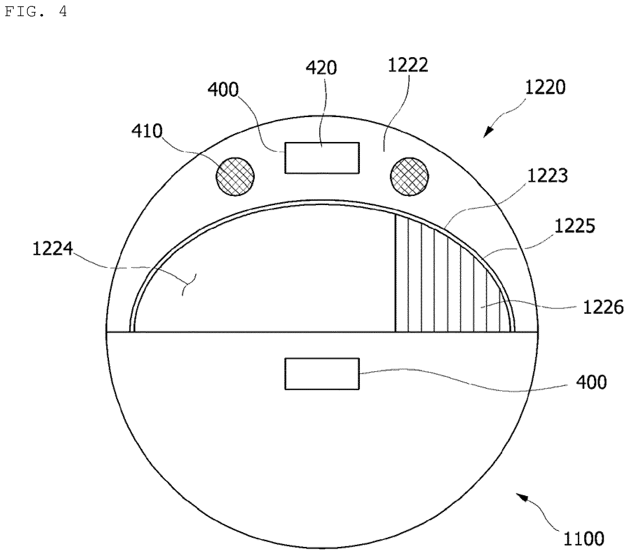

FIG. 3 is a plan view of the bed 1100 shown in FIG. 1, while FIG. 4 is a side view of the capsule-type phototherapy apparatus 1000 shown in FIG. 1.

Referring to FIG. 1, the capsule-type phototherapy apparatus (hereinafter referred to as 'the phototherapy apparatus') 1000 includes the bed 1100 and the cover 1200 for closing the bed 1100, wherein the bed 1100 and the cover 1200 have a space to accommodate a user therein.

The bed 1100 has a top open surface and is provided with a heat source inside the same, while the cover 1200 is pivotally installed at one side of the bed 1100 and includes a visible light emission device 1221 provided inside the same.

More particularly, the cover 1200 may be pivotally mounted on the bed 1100 by a hinge part 1410 fitted at an end of a control booth 1400 which will be described later.

Referring to FIG. 2, the cover 1200 comprises an upper cover 1210 to cover the head of a user or the body of the user corresponding to the precordial region or more, and a lower cover 1220 to cover the rest of the body other than the region covered by the upper cover 1210.

The upper cover 1210 has a curved transparent glass to entirely shield UV and may be fixed to the lower cover 1220 or detachably mounted on the same.

As another example, the upper cover 1210 may be made of a glass to transmit both UV A and UV B in order to facilitate generation of vitamin D by the user who receives phototherapy.

As another example, the upper cover 1210 may be made of a glass to block only UV B while transmitting UV A, thereby conducting a tanning procedure.

Although not shown in the drawings, an oxygen generator is installed at one side in the bed 1100 or the cover 1200. Further, the upper cover 1210 and the lower cover 1220 may cover the top surface of the bed 1100 and close the phototherapy apparatus 1000, so as to prevent bacteria, mold and dust from propagating over air to the outside while injecting clean and fresh oxygen from the oxygen generator into the phototherapy apparatus 1000, thereby implementing a clean room.

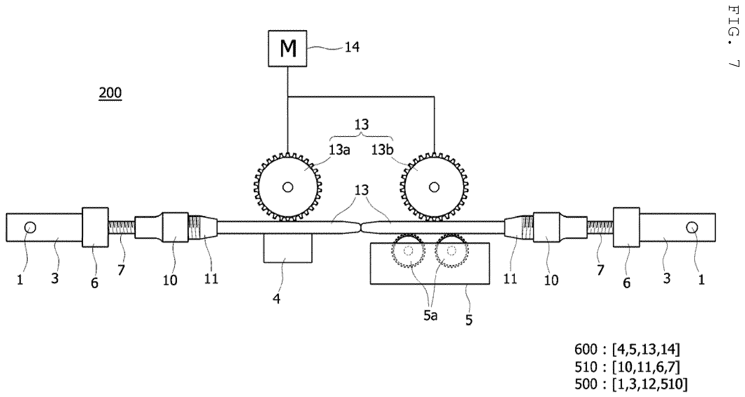

The visible light emission device 1221 may comprise a carbon rod 12 (shown in FIG. 7) provided therein, and conduct a procedure using visible light emitted by combustion of the carbon rod 12.

As another example, the tanning procedure may be executed for the user using UV A radiated by combustion of the carbon rod 12 above.

Configurations and operation of the visible light emission device 1221 will be concretely described later.

Referring to FIGS. 1 and 3, the heat source 1110 may be a compound based on rare elements and, more particularly, may be formed by combining rare elements such as germanium, red clay, mugwort, selenium, carbon, etc.

The heat source 1110 made of the compound may be heated by a heating plate provided on a bottom face of the heat source, so as to emit far-infrared light.

The bed 1100 and the cover 1200 may have an inner space formed in a streamline shape, and a reflection unit 1300 may be provided wholly or partially to the inner faces of the bed and the cove.

The reflection unit 1300 may include a bed side reflector 1310 and a cover side reflector 1320, wherein the far-infrared and visible light emitted from the heat source 1110 and the visible light emission device 1221, respectively, are reflected by the reflection unit 1300 to afford three-dimensional reflection about 360 degrees inside the phototherapy apparatus 1000, thereby implementing the procedure with the body of the user as a whole.

The lower cover 1220 may be provided with shower nozzles 1228 on the inner bottom face, wherein wound dressing may be conducted by spraying alcohol over the body of the user through the shower nozzles 1228. Moreover, shower or dry or wet sauna may be conducted.

Although not shown in the drawings, an aroma tank and a fumigator are provided at one side of the phototherapy apparatus 1000, and an aromatherapy procedure may be executed by ejecting heated and fumed aroma to the user through the shower nozzles 1228.

The lower cover 1220 may further include a blower 1229 at the inner bottom face, wherein internal ventilation of the sealed phototherapy apparatus 1000 is performed through the blower 1229.

In order for the user to be lying in a comfortable position during treatment, the bed 1100 may further include a mesh mat 1120 disposed on top of the bed.

The mesh mat 1220 may consist of a bio-mesh 1121 and a mesh frame 1122 for fixing the bio-mesh 1121, wherein both of the bio-mesh 1121 and the mesh frame 1122 have undergone antibacterial treatment.

The mesh mat 1120 is characterized in that visible and far-infrared light may pass through a space between lattices since the bio-mesh 1121 is arranged in a lattice-like form, thereby enabling far-infrared radiation and visible light treatment of the user's body as a whole.

The bed 1100 may have a seating part 1130 formed to protrude along the inner face on top of the bed, and the mesh mat 1120, more particularly, the mesh frame 1122 may be placed and fixed on the seating part 1130.

Since the mesh frame 1122 is stably secured to the seating part 1130, it is possible to prevent the mesh mat 1120 from being separated from the phototherapy apparatus 1000, for example, due to an overweight user.

The bed 110 may have a drain section 1140 on the bottom face and, after cleaning the inside of the bed 1100 and the cover 1200, sewage including foreign matter may be drained to the outside of the phototherapy apparatus 1000 through the drain section 114.

A control booth 1400 may be provided in the bed 1100 or at one side of the cover 1200.

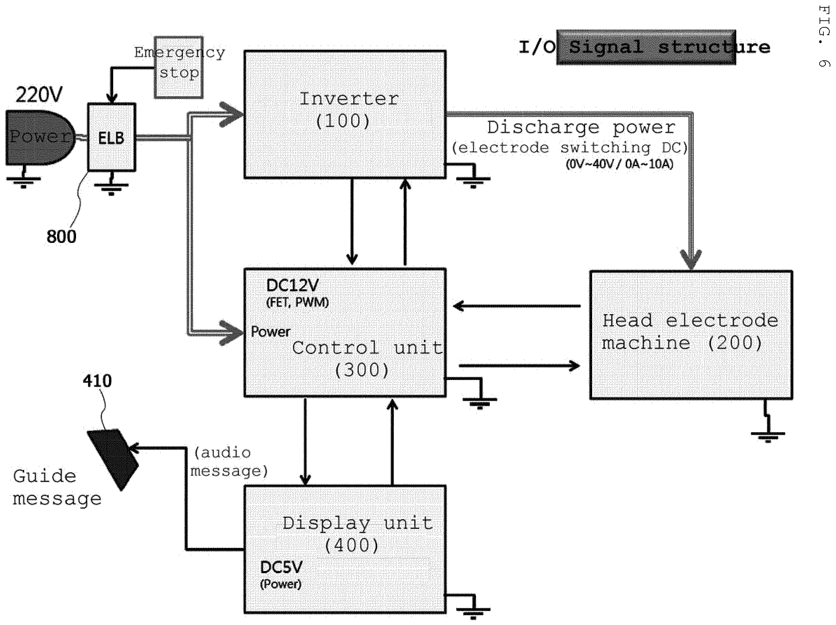

The control booth 1400 may include a motor 14 (shown in FIG. 7) to regulate opening/closing of the cover 1200 and adjust a spacing between the carbon rods 12, an inverter 100 (shown in FIG. 6), and a control unit 300 (shown in FIG. 6) to control operation of the phototherapy apparatus 1000, which are installed in the control booth.

In other words, a configuration for operation of the phototherapy apparatus 1000 is constructed inside the control booth 1000 rather than the outside of the phototherapy apparatus 1000, therefore, the phototherapy apparatus 1000 may be simplified and have improved aesthetic effects.

Referring to FIG. 4, the lower cover 1220 is opposite to the upper cover 1210 and has a separation part 1222 bent downward to the bed 1100, wherein the separation part 1222 comprises an incision portion 1223 which corresponds to or is cut wider than an area around the neck of the user or around the upper body of the user corresponding to the precordial region or more.

A through-hole 1224 is formed through the above incision portion 1223, wherein the user may lie between the upper cover 1210 and the lower cover 1220.

The incision portion 1223 may comprise a curtain rail 1225 and a blackout-reflecting curtain 1226 installed on the curtain rail 1225 along a longitudinal direction thereof.

The blackout-reflecting curtain 1226 may protect the user from glare due to visible light emitted from the visible light emission device 1221, and also reflect the visible light toward the body of the user.

On an outer face of the separation part 1222, a display unit 400 and an audio device 420 may be provided. Further, overall operation of the phototherapy apparatus 100 may be controlled through the display unit 400.

The display unit 400 may be a TFT LCD integrated with a touch pad 410, wherein conditions of the phototherapy apparatus 1000 including the heat source 1110 and the visible light emission device 1221 are illustrated on the display unit and the phototherapy apparatus 100 may be operated by touch of the user or an apparatus manager.

The display unit 400 may be mounted on the outer face of the separation part 1222 so that the user touches the display unit 400 while lying in the mesh mat 1120, thereby manipulating the phototherapy apparatus 1000.

In order for the manager to operate the phototherapy apparatus 1000 from the outside, an alternative display unit 400 may be additionally installed at one side of the bed 1100.

The audio device 420 may be mounted on both sides of the separation part 1220 to the head of the user, and transmit information on states of the phototherapy apparatus 1000 to the user via a guide message.

The audio device 420 may be operated by the display unit 400, in particular, built-in sound source data in the audio device 420 or the display unit 400 may be reproduced and act such that sound encompasses the head of the user through the audio device 420.

Thus the user can proceed with the sound therapy while not feeling bored during the procedure.

Although not shown in the drawings, the phototherapy apparatus 1000 may further include a pulse meter which is worn on a finger or a wrist of the user in order to improve safety during the procedure.

The pulse meter may be mounted on the bed 1100 or at one side of the cover 1200. When measuring pulses during phototherapy, if the measured value is out of a normal range, the control unit 300 (shown in FIG. 6) may stop running of the phototherapy apparatus 1000 or send a critical alarm to the audio device 420 or a cell phone of a guardian.

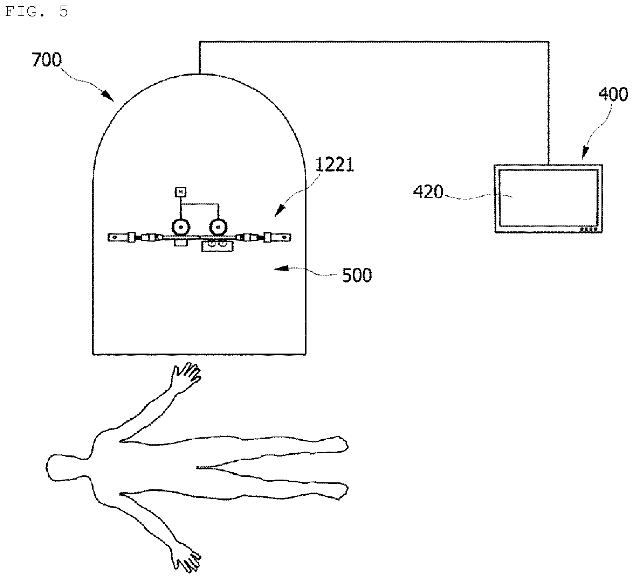

Hereinafter, with reference to FIGS. 5 to 12, the visible light emission device 1221 provided in the phototherapy apparatus 1000 will be described in detail.

FIG. 5 is a conceptual view illustrating the visible light emission device 1221 shown in FIG. 1, FIG. 6 is a block diagram illustrating the visible light emission device 1221 shown in FIG. 5, and FIG. 7 illustrates the visible light emission device 1221 shown in FIG. 5.

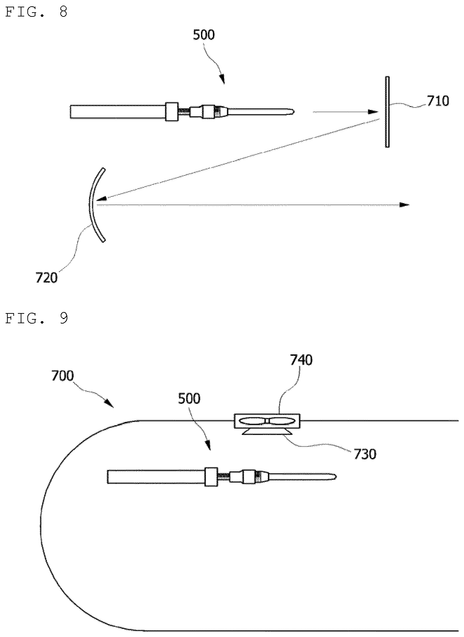

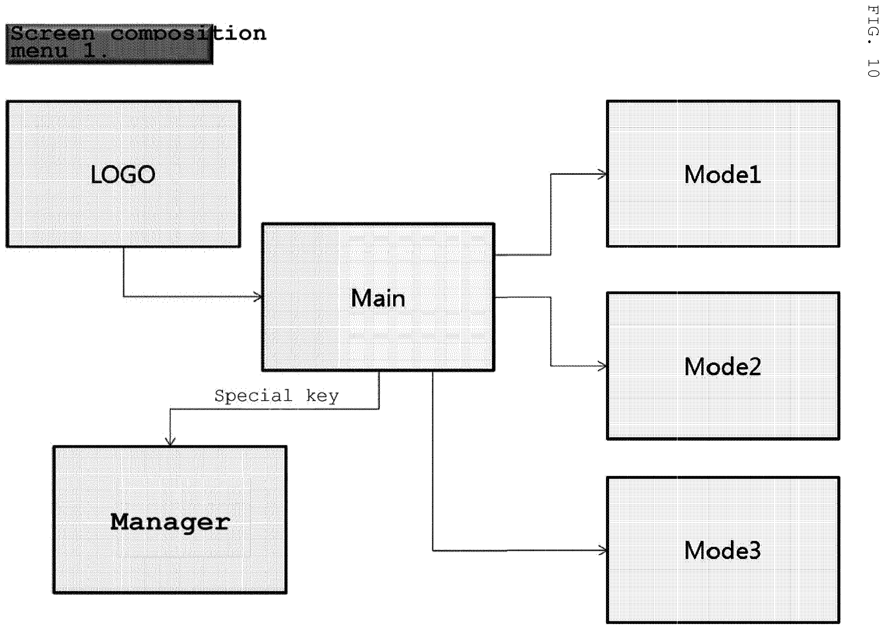

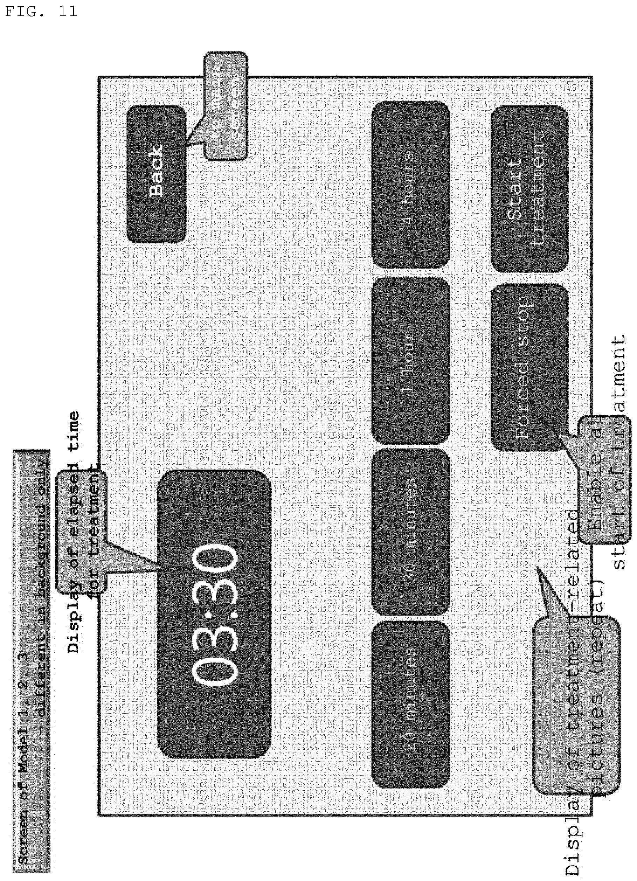

FIGS. 8 and 9 are schematic views of a cover unit shown in FIG. 5, FIG. 10 is a block diagram illustrating MODE of the display unit shown in FIG. 5, and FIG. 11 illustrates a time setting screen of the display unit shown in FIG. 5.

Referring to FIGS. 5 and 6, the visible light emission device 1221 comprises an inverter 100, a head electrode machine 200, as well as the control unit 300 and the display unit 400.

The inverter 100 may receive external power and convert the same into electric discharge power, and the head electrode machine 200 is configured to receive application of discharge power from the inverter 100 and then emit visible light to a user.

The control unit 300 may control the head electrode machine 200 and the inverter 100.

Accordingly, the control unit 300 may turn ON/OFF the discharge power generated from the inverter 100.

In addition, the control unit 300 may control voltage and current of the discharge power generated from the inverter 100.

Further, the control unit 300 may set time, that is, a timer, in order to maintain an intensity of the visible light for a predetermined period of time.

The display unit 400 may input a command signal to the control unit 300 and output states of the inverter 100 and the head electrode machine 200.

After all, if a user enters a command signal to the control unit 300 using the display unit 400, the control unit 300 may control the head electrode machine 200 and the inverter 100, thus emitting the visible light generated from the head electrode machine 200 to the user.

Further, when a command is received from the display unit 400, the control unit 300 may transmit the command to the inverter 100 and the head electrode machine 200 and output internal states of the inverter 100 and the head electrode machine 200 to the display unit 400 in real time.

More particularly, the control unit 300 may receive information indicating whether the discharge power is on/off from the inverter 100, state of the inverter 100, etc., and further receive the intensity of visible light, and states of machine components in the head electrode machine 200.

Therefore, the user can check in real time internal states of the inverter 100 and the head electrode machine 200, thereby efficiently responding to potential safety risk.

Referring to FIGS. 5 and 7, the head electrode machine 200 may include a pair of head electrodes 500, a spacing adjuster 600, a cover part 700 and a sensor part.

The pair of head electrodes 500 may be arranged at right and left sides to symmetrically face each other and have a carbon rod 12 built therein, while the spacing adjuster 600 is provided to adjust a distance between the pair of head electrodes 500.

Further, based on the distance between the pair of head electrodes 500, a voltage between the head electrodes 500 may be regulated.

Further, an optimum voltage for emitting visible light may range from 30 to 40 V. The farther the distance between the pair of head electrodes 500, the more increased the voltage. On the contrary, the nearer the distance between the pair of head electrodes 500, the decreased the voltage. That is, the distance between the pair of head electrodes 500 is proportional to the voltage. After all, when the head electrodes completely contact each other, the voltage may become 0 V.

The cover part 700 may be formed to be open at one face thereof in order to concentrically emit the visible light on desired body sites of the user where the head electrodes 500 and the spacing adjuster 600 are installed inside the cover part.

The sensor part is provided in the cover part 700 in order to sense an internal temperature of the cover part 700, visible light, and a voltage between the pair of head electrodes 500.

For these reasons, the sensor part may include a PT sensor, a UV sensor, an infrared sensor, a sensor for sensing an intensity of visible light, a detection circuit for detecting voltage, or the like.

Therefore, when the sensor part senses the intensity of visible light and/or a voltage between the pair of head electrodes 500 and transmits the sensed result to the control unit 200, the control unit 200 may output the result to the display unit 400, thereby informing the user of the present states of the above units.

In addition, as the carbon rod 12 becomes smaller while generating visible light for a long time, the voltage between the head electrodes 500 may be varied and thus may not maintain optimum visible light conditions.

Accordingly, when the sensor part senses fluctuation of voltage and transmits a state signal to the control unit 300 in order to maintain an optimum visible light emitting state, the control unit 300 may actuate the spacing adjuster 600 to decrease or increase a distance between the head electrodes 500, so as to automatically keep the voltage between the head electrodes 500 in a range of 30 to 40 V.

As a result, even though a length of the carbon rod 12 is reduced, the user can automatically maintain the optimum visible light emitting state without any particular operation until the carbon rod 12 is fully exhausted or for desired time.

In addition, a maximum distance between the head electrodes 500 may be appropriately set by the control unit 300.

Further, the user may control the discharge power generated from the inverter 100 through the control unit 300, thus emitting the visible light with desired intensity and wavelength. Further, adjusting the distance through the spacing adjuster 600 may maintain specific intensity and wavelength of the visible light desired by the user until the carbon rod 12 is fully exhausted.

Further, before applying the discharge power to the visible light emission device 1221 or when visible light emission is terminated, the control unit 300 may control the spacing adjuster 600 in order to widen the distance to the maximum value.

According to the adjustment as described above, the user may recognize that the visible light emission is terminated and the discharge power is not applied anymore. Therefore, it is possible to more stably replace the carbon rod 12 with a new one or to use the above visible light emission device 1221.

Each of the head electrodes 500 may include a carbon rod fixing member 510 and a conductive rod 3.

The conductive rod 3 may have a power connector 1 to which the discharge power is applied, while the carbon rod fixing member 510 is coupled at one side of the conductive rod 3 and is formed such that the carbon rod 12 is inserted and fixed in the fixing member 510.

The conductive rod 3 and the carbon rod fixing member 510 are formed such that electric current flows along the same, and thus, the discharge power can be applied to the carbon rod 12.

Accordingly, when the discharge power is applied to the conductive rod 3, current flows and reaches the end of the carbon rod 12, and visible light is generated in a spacing between a pair of carbon rods 12 inserted and fixed in the carbon rod fixing member 510. Further, an intensity of the visible light may be regulated depending upon a magnitude of the current.

Further, if the user inputs a command signal to the display unit 400 in order to regulate the intensity of the visible light, the control unit 300 may control the inverter 100 and regulate the discharge power, so as to increase or decrease the current.

As a result, since the intensity of the visible light can be regulated, the user may receive a desired intensity of visible light.

The carbon rod fixing member 510 may further include a suspension bolt 7, a first insulator 6, a carbon rod insertion part 11 and an insertion part-fixing nut 10.

The first insulator 6 may be disposed at a position at which the first insulator is coupled to the conductive rod 3, so as to prevent heat generated along the spacing between the pair of carbon rods 12 from being transferred toward the conductive rod 3, thereby protecting an electric wire connected to a terminal of the power connector 1 or other devices against influence of the heat.

Therefore, the first insulator 6 may prevent a lifespan of the visible light emission device 1221 from being shortened.

The carbon rod insertion part 11 is formed to allow a part of the carbon rod 12 to be inserted therein, and may have a screw thread formed on a part of an outer peripheral face thereof.

The insertion part-fixing nut 10 may be fastened to the screw thread of the carbon rod insertion part 11.

Briefly, since the carbon rod 12 is inserted into the carbon rod insertion part 11 and the insertion part-fixing nut 10 is fastened to the screw thread of the carbon rod insertion part 11, the carbon rod 12 is not separated from the carbon rod insertion part 11 even if the head electrodes 500 are moved by the spacing adjuster 600.

The suspension bolt 7 may be made of a conductive material and pass through the inside of the insulator 6, wherein one end of the bolt is inserted and fixed inside the conductive rod 3 while the other end is coupled in the carbon rod insertion part 11 to provide electric current to the carbon rod 12.

The spacing adjuster 600 may include a conveyer member 13, a pedestal member 4, a conveyer guider 5 and a motor 14.

The conveyer member 13 is disposed on top of a pair of carbon rods 12 which are symmetrically facing each other. More particularly, a first conveyer member 13a is disposed on top of the carbon rod 12 at the left side while a second conveyer member 13b is disposed on top of the carbon rod 13b at the right side, with reference to FIG. 7.

The conveyer member 13 may rotate while the outer peripheral face of the conveyer member is engaged with an outer peripheral face of the carbon rod 12, so as to move the carbon rod 12 from side to side.

The pedestal member 4 has a configuration such that it is disposed on the bottom of the carbon rod 12 at the left side, may prevent the carbon rod from escaping outside by unspecified external force when the carbon rod 12 is moved.

The conveyer guider 5 is disposed on the bottom of the carbon rod 12 at the right side and may have a plurality of rollers 5a installed therein.

The conveyer guider 5 may involve functions of the pedestal member 4 and be configured to facilitate movement of the carbon rod 12 by rotation of the conveyer member 13.

The motor 14 is installed at one side of the conveyer member 4 to rotate the first conveyer member 13a and the second conveyer member 13b.

In other words, the first conveyer member 13a may rotate forward while the second conveyer member 13b may have reverse rotation by the motor 14, resulting in narrower spacing between the carbon rods 12. On the contrary, when the first conveyer member 13a has reverse rotation while forward rotating the second conveyer member 13b, the spacing between the carbon rods 12 increases.

For this reason, a rotation direction of the conveyer member 13 may alter the spacing between the carbon rods 12 to cause voltage fluctuation.

Referring to FIGS. 7 and 8, the cover part 700 may include a first reflector 710 and a second reflector 720.

The cover part 700 is provided inside the lower cover 1220 shown in FIG. 1, and the first reflector 710 is mounted on a part of an open face of the cover part 700 to reflect visible light toward an inner peripheral face of the cover part 700.

On the other hand, the second reflector 720 is mounted on the inner peripheral face of the cover part 700 and may reflect the visible light already reflected by the first reflector 710 to the open face so as to forward the visible light to the outside, thereby irradiating an affected portion of a patient ('lesion') with the reflected light.

If the first reflector 710 and the second reflector 720 are not provided, the visible light generated by the carbon rod 12 is not emitted in a constant direction, that is, in a straight line, but may spread in all directions. Further, NULL point at which a shadow is generated by the head electrodes 500 may possibly exist and inhibit efficient emission of visible light.

Therefore, the first reflector 710 and the second reflector 720 may efficiently emit the visible light in desired direction.

Further, the first reflector 710 and the second reflector 720 may have different sizes and/or shapes on the basis of design of the visible light emission device 1221.

Further, the first reflector 710 and the second reflector 720 may be formed with the same structure as a headlamp of a vehicle.

The cover part 700 may further include a funnel duct 730 formed to discharge combustion gas generated by the pair of carbon rods 12 to the outside; and a ventilation fan 740 to efficiently intake the combustion gas in the funnel duct 730.

Further, in order to prevent foreign materials from entering the ventilation fan 740, a filter may be mounted on top of the ventilation fan 740.

Further, the filter may be mounted on the cover part 700 along with the ventilation fan 740 or separately.

The filter may filter air pollutants generated from the carbon rod 12 in order to prevent the air pollutants from being inhaled by the user before discharging the same out of the cover part 700.

On the open face of the cover part 700, a wire mesh for shielding electromagnetic radiation generated in the apparatus may be further provided.

The wire mesh is grounded to the cover part 700 and may shield the electromagnetic waves to prevent the same from being discharged outside if the electromagnetic radiation is generated.

The audio device 410 and the touch pad 420 may be included in the display unit 400.

The audio device 410 may be formed to output an audio message to the user in relation to information on the state of the carbon rod 12 detected by the control unit 300.

The user or the manager may press the touch pad 420 and send a command signal to the control unit 300.

Meanwhile, referring to FIG. 10, a command signal for emitting visible light may be simply defined as MODE1, MODE2 and MODE3 for user convenience.

The MODE1 transmits a command signal to the control unit in order to regulate the discharge power of the inverter 100 and irradiate the user with a low intensity of visible light.

The MODE2 transmits another command signal to the control unit in order to regulate the discharge power of the inverter 100 and irradiate the user with a high intensity of visible light.

The MODE3 transmits a further command signal to adjust a distance between a pair of head electrodes 500 or regulate the discharge power of the inverter 100, thereby enabling fluctuation of intensity and wavelength of the visible light.

Further, a command signal for setting a visible light emission time may be set, as shown in FIG. 7.

Referring to FIG. 6, the visible light emission device 1221 may further comprise an emergency stop switch 800.

The emergency stop switch 800 may be provided between the inverter 100 and an external power source in order to block the external power.

Therefore, pressing the emergency stop switch 800 may block the power supplied to the inverter 100 to cut off the discharge power generated by the inverter 100.

Accordingly, if the user is in an emergency situation or the visible light emission device 1221 malfunctions due to overcurrent, operation of the visible light emission device 1211 can be instantly stopped by pressing the emergency stop switch 800.

Further, the emergency stop switch 800 may take the form of an earth leakage breaker (ELB).

Further, a chute switch of an output terminal of the ELB may be provided at the outside and configured to block both of input and output parts when pressing the switch.

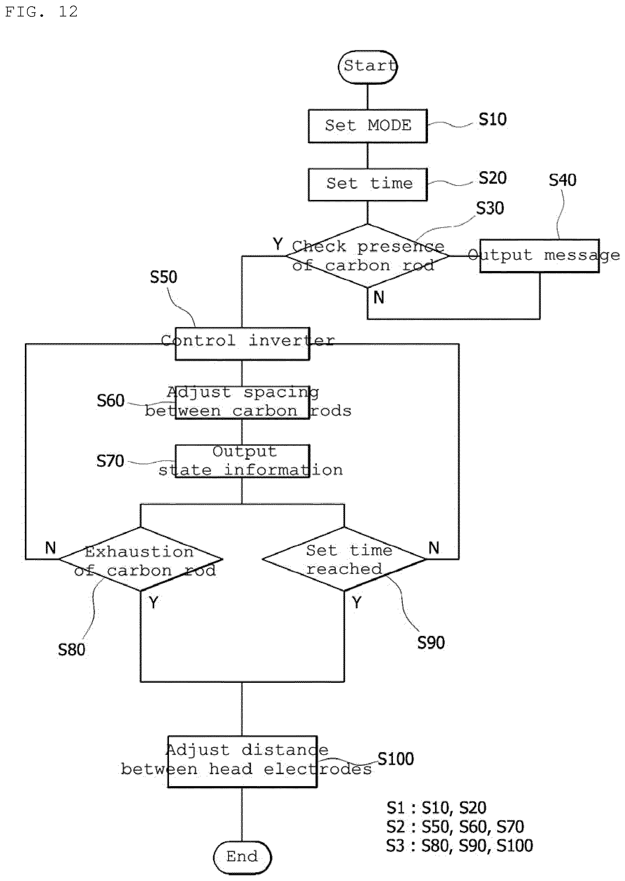

With reference to FIG. 12, a light emitting method by the visible light emission device 1221 will be described in detail below.

FIG. 12 is a flowchart illustrating a process of emitting light by the visible light emission device 1221 shown in FIG. 5.

The light emitting method may include a first step for setting a command signal S1, a second step for checking whether the carbon rod 12 has been inserted or not S30, a third step for emitting visible light S2, and a fourth step for terminating emission of the visible light S3.

The first step S1 may set a command signal to be transmitted to the control unit 300 via the touch pad 420 of the display unit 400.

The second step S30 may use the control unit 300 and check whether the carbon rod 12 has been inserted in the carbon rod fixing member 510 or not.

The third step S2 may emit visible light from the head electrode machine 200 according to the command signal set by the control unit 300.

The fourth step S3 may terminate emission of visible light.

The first step S1 may include a mode setting stage 1-1 (S10) and a time setting stage 1-2 (S20).

The stage 1-1 (S10) may set one of multiple modes wherein the intensity of emitted visible light is set by levels.

The set modes may include MODE1, MODE2 and MODE3 as described above.

The stage 1-2 (S20) may set a visible light emission time.

The time may be set by a timer in the control unit 300 and the user may emit the visible light for a desired period of time.

The second step S30 may include stage 2-1 for outputting a message to request replacement or insertion of the carbon rod 12 to the display unit (S40) when presence of the carbon rod 12 in the carbon rod fixing member 510 is not sensed.

The third step S2 may include stage 3-1 for controlling the inverter 100 (S50), stage 3-2 for adjusting a spacing between a pair of carbon rods 12 (S60), and stage 3-3 for outputting a state signal (S70).

The stage 3-1 (S50) may control the inverter 100 according to the command signal set in the first step S1.

The stage 3-2 (S60) may operate the spacing adjuster 600 according to the command signal set in the first step S1 in order to adjust a spacing between the pair of carbon rods 12 and emit visible light.

The stage 3-3 (S70) may output the state of the inverter 100, and intensity condition and a temperature of the emitted visible light to the display unit 400.

The fourth step S3 may include stage 4-1 for checking full exhaustion of the carbon rod 12 (S80) and stage 4-3 for adjusting a distance between the head electrodes 500 (S100).

If full exhaustion of the carbon rod 12 is determined in the stage 4-1 (S80), the control unit 300 may control the spacing adjuster 600 in order to adjust a distance between the head electrodes 500 to the maximum value in the stage 4-3 (S100).

Further, before operation of the spacing adjuster 600, the control unit 300 may transmit a control signal or command signal to turn OFF the discharge power to the inverter 100 for user safety.

Further, if it is checked that the carbon rod 12 has not been fully exhausted in stage 4-1 (S80),the process feedbacks to the second step S30.

The maximum distance between the head electrodes 50 may be set by the control unit 300.

Therefore, the process is continuously fed back to the second step S30 until the carbon rod 12 is fully exhausted.

Accordingly, if it is identified that the carbon rod 12 has been fully exhausted, the control unit 300 may control the spacing adjuster 600 in order to adjust the distance between the head electrodes 500 to the set maximum value.

The fourth step S3 may include stage 4-2 for checking whether to reach the set time (S90), and stage 4-3 for adjusting the distance between the head electrodes 500 (S100).

The stage 4-2 (S90) may determine whether to reach the set time.

Upon determining that the set time has been reached in stage 4-2 (S90), the control unit 300 may control the spacing adjuster 600 in order to adjust the distance between the head electrodes 500 to the maximum value in stage 4-3 (S100).

In addition, before operation of the spacing adjuster 600, the control unit 300 may transmit a control signal to turn OFF the discharge power to the inverter 100 for user safety.

Further, upon determining that the set time has not been reached in stage 4-2 (S90), the process feeds back to the second step S30.

As described above, it is possible to attain effects of executing far-infrared and visible light treatment procedures to the body of a user by the capsule-type phototherapy apparatus 1000 according to an embodiment of the present invention.

Further, it is possible to attain effects of implementing a clean room with prevention of bacteria, mold and dust propagated over air to the outside by the bed 1100 and the cover 1200 described above.

Further, it is possible to attain effects of enabling the user to receive treatment in a pleasant or comfortable environment through functions of constant temperature and humidity, shower, dry and wet sauna, music therapy and aromatherapy.

Preferred embodiments of the present invention have been described in the above detailed description as well as the drawings. Although specific terms have been used herein, these are only for the purpose of illustrating the present invention but are not intended to particularly limit their meanings or the scope of the present invention as set forth in the appended claims. Therefore, those skilled in the art will appreciate that various modifications and/or equivalent embodiments are possible from the above disclosure. Accordingly, the technical configurations of the present invention to be protected should be defined by the technical spirit of the appended claims.

## Claims

1. A capsule-type phototherapy apparatus, comprising:
a bed in which a heat source is installed, wherein a user can lie on the top of the bed; and
a bed cover in which a visible light emission device is installed and which is rotatably installed at one side of the bed so as to open and close the bed.

2. The apparatus according to claim 1, wherein the bed comprises an antimicrobial treatment mesh mat disposed on the top thereof.

3. The apparatus according to claim 2,
the mesh mat comprising:
a bio-mesh in a lattice form; and
a mesh frame for fixing the bio-mesh.

4. The apparatus according to claim 2, wherein the bed comprises a seating part formed to protrude along the inner face on the top of the bed, wherein the mesh mat is placed on the seating part.

5. The apparatus according to claim 1, wherein the bed comprises a display unit mounted on the outer face thereof.

6. The apparatus according to claim 1,
the cover comprising:
an upper cover to cover the head of the user or the body of the user corresponding to the precordial region or more; and
a lower cover to cover the rest of the body other than the region covered by the upper cover.

7. The apparatus according to claim 6, wherein the lower cover comprises a separation part bent downward to the bed.

8. The apparatus according to claim 7, wherein the separation part comprises a display unit and an audio device installed on the outer face of the separation part.

9. The apparatus according to claim 7, wherein the separation part comprises an incision portion which corresponds to or is cut wider than an area around the neck of the user or around the upper body of the user corresponding to the precordial region or more.

10. The apparatus according to claim 9, wherein the incision portion comprises a curtain rail and a blackout-reflecting curtain installed on the curtain rail along a longitudinal direction thereof.

11. The apparatus according to claim 6, wherein the lower cover is provided with shower nozzles therein.

12. The apparatus according to claim 1, wherein the bed and the cover have inner spaces formed in a streamline shape, and a reflection unit is provided wholly or partially on the inner faces of the bed and the cover

13. The apparatus according to claim 1, wherein a control booth is provided at one side of the bed or the cover.

14. The apparatus according to claim 1,
the visible light emission device comprising:
an inverter for receiving external power and converting the same into electric discharge power; and
a head electrode machine provided with a carbon rod, receiving discharge power from the inverter and then emitting visible light to the user by combustion of the carbon rod.
